# FASCICULE DE BREVET EUROPEEN

(11) **EP 0 771 550 B1**
(45) Date de publication et mention de la délivrance du brevet: **12.03.2003**
(21) Numéro de dépôt: 97200129.1
(22) Date de dépôt: 07.11.1990
(51) Int. Cl.: A61F 2/01, A61M 25/00, A61M 25/04, B29C 65/02, B29C 71/02

(54) **Procédé pour la fabrication d'un filtre-cathéter, et filtre-cathéter**
Verfahren zur Herstellung eines Filter-Katheters, und Filter-Katheter
Process for manufacturing a filter-catheter, and filter-catheter

(30) Priorité: 13.12.1989 FR 8917201
(43) Date de publication de la demande: 07.05.1997
(62) Demande divisionnaire de: 90403150.7
(73) Titulaire: Lefebvre, Jean-Marie, 59800 Lille (FR)
(72) Inventeur: Lefebvre, Jean-Marie, 59800 Lille (FR)
(74) Mandataire: 't Jong, Bastiaan Jacob

(56) Documents cités:
- EP-A- 0 307 871
- FR-A- 2 213 146
- FR-A- 2 343 488
- FR-A- 2 606 642
- GB-A- 955 490
- US-A- 4 425 908
- US-A- 4 832 055

## Description

La présente invention concerne un procédé pour la fabrication d'un filtre-cathéter comme décrit dans le préambule de la revendication 1.

Un filtre-cathéter fabriqué avec un tel procédé est connu du brevet FR-A-2 606 642.

Pour la mise en condition active du filtre, c'est-à-dire pour déformer les bandes flexibles, vers leur état déployé, la canalisation est déplacée dans la direction distale sur le conduit intérieur. Par conséquent la canalisation est soumise à des forces de compression. Cette canalisation doit être dimensionnée de telle sorte que celui-ci soit résistant à ces forces.

La présente invention vise à procurer un procédé comme décrit dans l'introduction, selon lequel un filtre-cathéter amélioré peut être fabriqué.

Ce but est atteint avec la procédure selon l'invention par les mesures citées dans la caractéristique de la revendication 1.

Il est à noter que de US-A-4 832 055 un filtre est connu, destiné à être déployé dans un vaisseau sanguin. Le filtre comprend plusieurs fils préformés dans une forme non déployée et tordue. Les fils sont métalliques et tordus pour être capable d'adopter une forme aplatie quand ils sont déployés.

L'invention concerne et fournit aussi un filtre-cathéter comme indiqué à la revendication 2.

D'autres caractéristiques et avantages ressortiront plus clairement de la description qui va être faite du mode préféré de réalisation du filtre-cathéter à usage temporaire et de son procédé de fabrication, illustré par les dessins annexés dans lesquels:
- La figure 1: est une vue schématique en coupe du filtre au repos;
- La figure 2: est une vue schématique en coupe du filtre déployé;
- La figure 3: est une vue en coupe transversale du filtre selon l'axe D de la figure 2;
- La figure 4: est une vue schématique en coupe de l'extrémité distale d'un filtre;
- La figure 5: est une vue schématique de côté du dispositif de maintien du filtre-cathéter pendant le traitement de thermofixation.

Le filtre-cathéter 1 de l'invention se compose essentiellement d'une canalisation 2 creuse et d'un conduit creux 3 qui est placé coaxialement à l'intérieur de la canalisation 2. Ces deux éléments, canalisation 2 et conduit 3 sont en polytétrafluoréthylène.

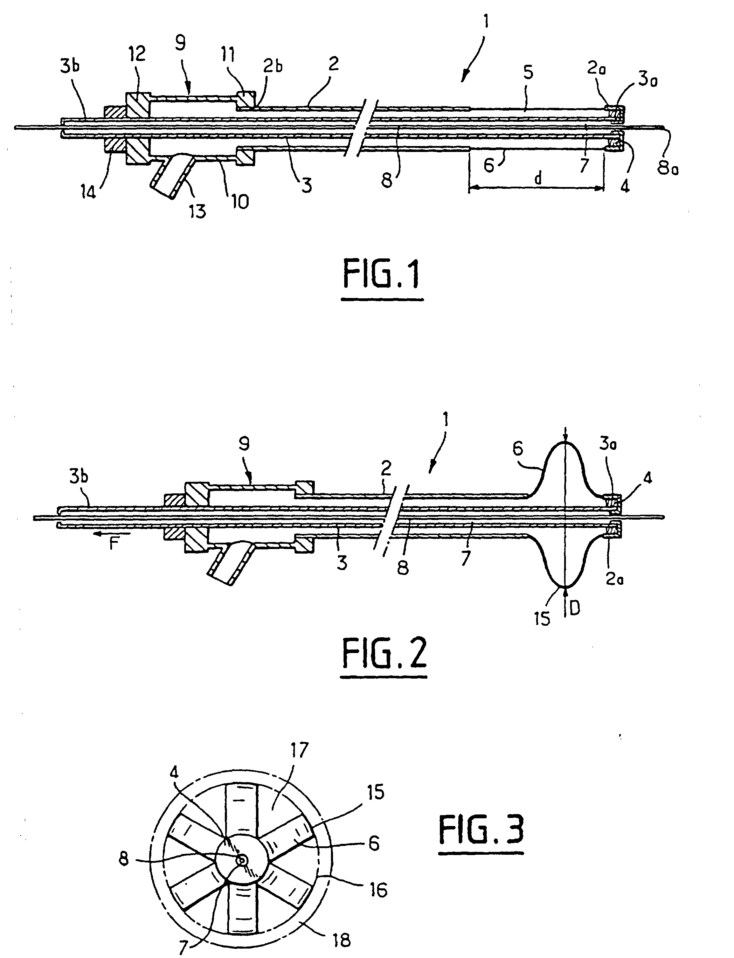

Dans le présent texte les termes "proximal" et "distal" sont employés en prenant pour référence la partie du corps du patient par où le filtre est introduit. Ainsi l'extrémité distale du filtre, représentée à droite sur les figures 1 et 2, est l'extrémité la plus éloignée de cette partie du corps, et l'extrémité proximale la plus rapprochée.

Les extrémités distales respectivement 2a de la canalisation 2 et 3a du conduit 3 sont thermosoudées ensembles et autour d'un anneau métallique 4, en acier inoxydable, platine ou or. Cet anneau 4 est dans un matériau radio-opaque, détectable lors du positionnement du filtre.

Comme cela ressort clairement des figures 1 et 2, l'anneau 4 est placé entre la canalisation 2 et l'extrémité distale 3a du conduit 3, l'extrémité distale 2a de la canalisation étant repliée à l'intérieur du conduit 3 et recouvrant l'extrémité distale 3a dudit conduit. Le thermosoudage réalise l'assemblage de ces éléments pour former l'extrémité 15 du filtre-cathéter.

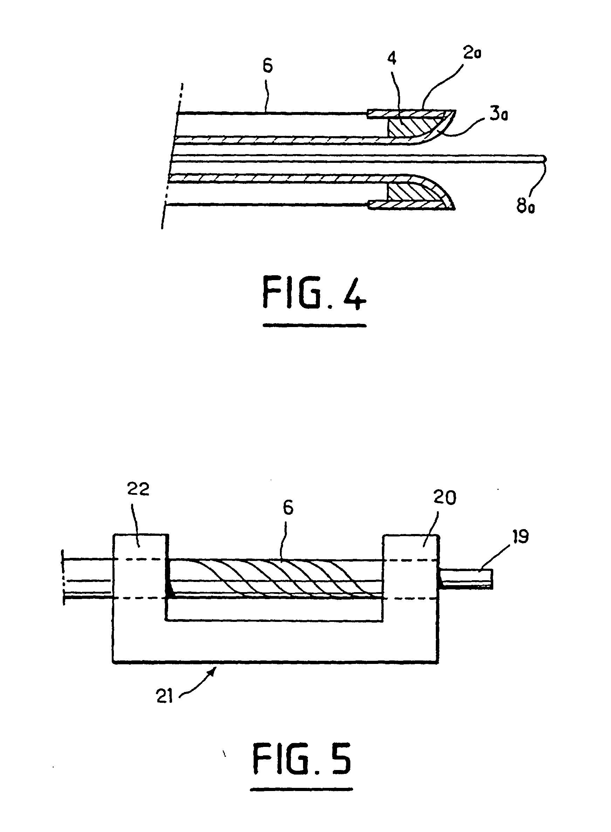

Selon un autre mode de réalisation illustré à la figure 4, le thermosoudage intervient alors que l'extrémité distale 3a du conduit intérieur 3 déborde au-delà de l'anneau métallique 4 et s'évase vers l'extérieur venant au contact de l'extrémité distale 2a de la canalisation 2.

En amont de son extrémité distale 2a, la canalisation 2 comporte six entailles 5, pratiquées dans le sens longitudinal sur une longueur d de 30 mm; elles sont régulièrement réparties sur la périphérie de la canalisation. Ces entailles 5 délimitent six bandes 6 identiques, chacune d'elles occupe un espace angulaire de 60° de la canalisation 2. Pour une canalisation 2 de 3mm de diamètre, chaque bande 6 a une largeur d'environ 1,5mm.

Le conduit 3 est creux et comporte un canal central 7, dans lequel peut librement coulisser une tige métallique 8 servant de tige de guidage lors de l'introduction du filtre-cathéter.

Vers l'extrémité proximale, mais à l'extérieur du corps du patient, la canalisation 2 est fixée sur un dispositif 9 de maintain et d'alimentation. Ce dispositif 9 comporte une tubulure cylindrique et des baques 11 et 12 d'extrémité. La première baque 11 annulaire raccorde l'extrémité proximale 2b de la canalisation 2 à la partie avant de la tubulure 10. La seconde baque 12 entoure le conduit 3 et ferme la partie arrière de la tubulure 10. Un coude 13 débouche en oblique dans le corps de la tubulure 10.

L'extrémité proximale 3b du conduit 3 va au-delà de la tubulure 10. La baque 12 enserre cette extrémité distale 3b de façon étanche; toutefois le conduit 3 peut coulisser longitudinalement dans la baque 12 par rapport à la tubulure 10 et donc à la canalisation 2.

Une baque de blocage 14 est apte à assurer le blocage du conduit 3 contre la bague 12.

La tubulure 10 est fixée de manière connue à proximité du patient sur un bâti supportant deux systèmes d'alimentation de produits à injecter, débouchant pour le premier dans l'extrémité proximale 3b du conduit et pour le second dans le coude 13 de la tubulure 10.

La mise en oeuvre du filtre-cathéter se déroule dans les conditions suivantes.

Dans une première phase, la tige de guidage 8 est introduite par voie percutanée dans la veine adéquate, jusqu'à ce que son extrémité 8a distale se trouve en position dans la veine cave inférieure du patient.

Dans une deuxième phase, on fait coulisser depuis l'extérieur du corps du patient le filtre-cathéter 1 le long de la tige de guidage 8 jusqu'à ce que l'anneau 4 se trouve en position dans la veine cave, là où le filtre doit être implanté.

Pendant le déroulement de cette deuxième phase, les bandes 6 sont au repos, c'est-à-dire qu'elles sont dans le prolongement de la canalisation 2.

Dans une troisième phase illustrée par la figure 2 et correspondant au déploiement du filtre, la baque de blocage 14 est desserrée en sorte de permettre au conduit 3 de coulisser dans la bague 12. Puis on déplace avec précaution l'extrémité proximale 3b du conduit 3 vers l'arrière dans le sens de la flèche F. Dans cette phase, tout le conduit 3 se déplace ainsi que l'anneau 4, solidaire de l'extrémité distale 3a du conduit. Etant donné que la tubulure 10 et donc la canalisation 2 sont maintenues en position, le recul de l'anneau 4 dans le sens de la flèche F ne peut se produire que grâce à la présence des bandes 6 flexibles. Chaque bande 6 se plie individuellement, créant de ce fait une deformation transversale homogène de la canalisation dans la zone des entailles 5. Le déplacement et la longueur des bandes 6 sont choisis de telle sorte que, en position de déploiement, les parties médianes 15 des bandes 6, correspondant au plan D de courbure, prennent appui sur la paroi interne 16 de la veine. Une fois que le filtre est correctement déployé, la baque de blocage 14 est resserrée en sorte d'empêcher tout déplacement du conduit 3 par rapport à la canalisation 2 et donc de maintenir les bandes 6 dans leur état de déformation transversale.

Comme cela apparaît clairement sur la figure 3, qui est une coupe selon le plan D, les six bandes 6 sont déployées transversalement par rapport à la canalisation 2 et obturent partiellement l'intérieur 17 de la veine 18. Ainsi les caillots sanguins se déplaçant à l'intérieur 17 de la veine 18 sont arrêtés par les bandes 6 tandis que le sang peut circuler librement.

Préalablement à l'utilisation du filtre-cathéter, on a fait subir à celui-ci un traitement thermique de thermofixation. Ce traitement a consisté à donner un choc thermique à la partie distale du filtre alors que les bandes flexibles 6 sont dans leur état déployé, comme illustré à la figure 2. La durée de ce traitement et la température sont déterminées en sorte que la forme prise par les bandes soit en quelque sorte mémorisée dans la structure polymérique du polytétrafluoréthylène. Ce traitement augmente la résistance à la déformation des bandes 6 et évite l'affaissement du filtre à l'intérieur de la veine 18 et le risque de thrombogénèse dû aux turbulences créées dans le flux sanguin par l'hétérogénéité de disposition des bandes 6 dans la veine.

Le traitement thermique est appliqué sur la partie distale du filtre-cathéter alors que les bandes flexibles 6 ont une forme torsadée (voir figure 5). Pour cela, on emmanche dans le conduit intérieur 3 un mandrin 19 de manière à rigidifier la partie distale du filtre-cathéter; on bloque en position l'extrémité distale 2a de la canalisation 2 dans la branche avant 20 d'un support de maintien 21, on fait subir à la canalisation 2 une rotation sur elle-même, qui confère aux bandes flexibles 6 la forme torsadée illustrée sur la figure 5, puis on bloque en position la canalisation 2 en amont desdites bandes flexibles 6 dans la branche arrière 22 du supports de maintien 21. Le support 21 et la partie distale du filtre-cathéter sont placés dans un four de cuisson par exemple par rayonnement micro-onde pour y subir le traitement thermique, pendant 4 à 5 minutes. On remarque que lors de l'utilisation du filtre, les bandes flexibles 6 ont tendance à prendre la forme en hélice conférée lors du traitement thermique.

Un produit de traitement thérapeutique peut être injecté en amont du filtre à travers le conduit 3, à partir de l'extrémité proximale 3b de celui-ci. Il s'agit d'un anticoagulant du type héparine ou d'un fibrinilotique. Le conduit 3 peut comporter des orifices, par exemple disposés en spirale, dans la zone faisant face aux bandes 6. Ainsi le produit de traitement thérapeutique introduit dans le conduit 3 peut être réparti à la fois en amont et au niveau du filtre lui-même. Le même produit, ou éventuellement un autre produit de traitement thérapeutique peut être injecté dans la zone immédiatement en aval du filtre à travers la canalisation 2, à partir du coude 13 de la tubulure 10.

Une fois le traitement jugé satisfaisant, le retrait du filtre s'effectue de la manière suivante. La baque de blocage 14 est desserrée et l'extrémité proximale 3b du conduit 3 est repoussée dans le sens inverse de la flèche F jusqu'à ce que les languettes 6 retrouvent leur position de repos, rectilignes et parallèles entre elles. Puis, la baque 14 est de nouveau resserrée, et l'ensemble conduit 3 et canalisation 2 est retiré de la veine.

Les essais réalisés à l'aide du filtre-cathéter selon l'invention ont permis de constater l'absence de toute formation de caillots sangains du fait de la présence du filtre dans la veine.

La présente invention n'est pas limitée au mode de réalisation qui a été décrit à la titre d'example non exhaustif, mais en couvre toutes les variantes qui découlent de la revendication 1. En particulier la tige de guidage 8 peut être creuse et permettre l'injection de produit de traitement en aval du filtre voire même bien au-delà de l'extrémité distale du filtre-cathéter 1.

## Revendications

1. Procédé pour la fabrication d'un filtre pour interrompre partiellement et au moins temporairement un vaisseau sanguin, comprenant la procuration d'une canalisation (2) avec une extrémité distale et une extrémité proximale, la mise dans la partie distale, symétriquement répartis sur la périphérie, d'entailles longitudinales délimitant des bandes flexibles (6), la procuration d'un conduit intérieur (3) aussi de polytétrafluoréthylène, avec une extrémité distale et une extrémité proximale, la mise dans la canalisation du conduit intérieur (3) et le solidarisation seulement aux extrémités distales (2a, 3a) de la canalisation (2) et du conduit (3) **caractérisé en** faisant déformer vers leur état-déployé les bandes flexibles délimitées par les entailles longitudinales, tordre en maintenant en torsion la partie distale comprenant les entailles de la canalisation, et en soumettant les bandes flexibles à un traitement de thermofixation d'une durée telle et d'une température telle que la forme déployée et tordue est mémorisée.

2. Filtre-cathéter produit par le procédé de la revendication 1 pour interrompre partiellement et au moins temporairement un vaisseau sanguin, comprenant une canalisation (2) comprenant dans sa partie distale, symétriquement répartie sur la périphérie, des entailles longitudinales délimitant des bandes flexibles (6) et un conduit intérieur (3) dans la canalisation (2) où la canalisation (2) et le conduit intérieur (3) sont solidarisés à leurs extrémités distales (2a, 3a) **caractérisé en ce que** les bandes flexibles (6) ont une forme déployée et tordue dans leur condition non-chargée, cette forme étant oblenue par thermofixation.

## Claims

1. Process for the manufacture of a filter to partially and at least temporarily interrupt a blood vessel, comprising procuring a pipe (2) with a distal end and a proximal end, placing in the distal part, symmetrically distributed around its periphery, longitudinal slots defining flexible strips (6), procuring an internal conduit (3) also of polytetrafluoroethylene, with a distal end and a proximal end, placing the internal conduit (3) in the pipe and firmly attaching only at the distal ends (2a, 3a) the pipe (2) and the conduit (3), **characterised by** deforming the flexible strips defined by the longitudinal slots into their deployed state, twisting while maintaining under torsion the distal part comprising the slots in the pipe, and subjecting the flexible strips to a heat fixing treatment of a duration such and of a temperature such that the deployed and twisted form is memorised.

2. Filter-catheter produced by the process of claim 1, to partially and at least temporarily interrupt a blood vessel, comprising a pipe (2) including in its distal part, symmetrically distributed around its periphery, longitudinal slots defining flexible strips (6) and an internal conduit (3) in the pipe (2) in which the pipe (2) and the internal conduit (3) are firmly attached at their distal ends (2a, 3a), **characterised by** the fact that the flexible strips (6) have a deployed and twisted form in their condition without load, this form being obtained by heat fixing.

## Patentansprüche

1. Verfahren zur Herstellung eines Filters zum teilweisen und zumindest zeitweisen Unterbrechen eines Blutgefäßes, mit dem Vorsehen eines Kanals (2) mit einem distalen Ende und einem proximalen Ende, dem Vorsehen im distalen Abschnitt von länglichen Einschnitten, die symmetrisch über den Umfang verteilt sind und die flexible Bänder (6) begrenzen, dem Vorsehen einer inneren Leitung (3), ebenfalls aus Politetrafluorethylen, mit einem distalen Ende und einem proximalen Ende, dem Anbringen der Innenleitung (6) in dem Kanal und der einzigen festen Verbindung an den distalen Enden (2a, 3a) des Kanals (2) und der Leitung (3), **dadurch gekennzeichnet, daß** die flexiblen Bänder, die durch die länglichen Einschnitte begrenzt sind, in deren aufgeweiteten Zustand verformt werden, daß der distale Abschnitt der die Einschnitte das Kanal aufweist, verdreht wird und in der Verdrehung gehalten wird und daß die flexiblen Bänder einer Heißfixierungsbehandlung über eine solche Dauer und bei einer solchen Temperatur unterworfen werden, das die aufgeweitete und verdrehte Form gespeichert wird.

2. Gemäß dem Verfahren nach Anspruch 1 hergestellter Filter-Katheter zum teilweisen und zumindest zeitweisen Unterbrechen eines Blutgefäßes, mit einem Kanal (2), der an seinem distalen Abschnitt symmetrisch über den Umfang verteilt längliche Einschnitte aufweist, die flexible Bänder (6) begrenzen, und einer inneren Leitung (3) in dem Kanal (2), wobei der Kanal (2) und die innere Leitung (3) an ihren distalen Enden (2a, 3a) fest miteinander verbunden sind, **dadurch gekennzeichnet, daß** die flexiblen Bänder (6) eine aufgeweitete und verdrehte Form im unbelasteten Zustand aufweisen, wobei die Form durch Heißfixierung erreicht ist.
